# EUROPEAN PATENT APPLICATION

(11) **EP 4 599 867 A1**
(43) Date of publication of application: **13.08.2025**
(21) Application number: 24156141.4
(22) Date of filing: 06.02.2024
(51) Int. Cl.: A61M 5/178

(54) **COLORANT FOR SILICONIZATION OF SYRINGES**

(71) Applicant: Becton, Dickinson and Company, Franklin Lakes, NJ 07417-1880 (US)
(72) Inventor: GONTARD, Lucile, 38760 Varces-Allieres-et-Risset (FR)
(74) Representative: Germain Maureau

(57) **Abstract**

The invention relates, in general, to a colorized oil comprising a combination of at least one oil (e.g., one or more silicone oils and/or one or more hydrocarbon oils) in combination with at least one colorization compound selected from one or more fluorescent compositions (e.g., one or more acrylic fluorescent compositions and/or one or more ultraviolet dyes).

## Description

### Field of the Invention

The invention relates, in general, to a colorized oil comprising a combination of at least one oil (e.g., one or more silicone oils and/or one or more hydrocarbon oils) in combination with at least one colorization compound selected from one or more fluorescent compositions (e.g., one or more acrylic fluorescent compositions and/or one or more ultraviolet dyes). In one embodiment, the present invention relates to a colorized oil comprising at least one oil selected from one or more silicone oils, one or more hydrocarbon oils, or a combination of any two or more thereof, where the at least one oil has viscosity in the range of about 500 centipoise to about 1500 centipoise, in combination with at least one colorization compound selected from one or more fluorescent acrylic compositions, one or more ultraviolet dyes, or a combination of any two or more thereof, where the at least one colorization compound fluoresces at a wavelength of about 100 nm to about 400 nm.

### Description of the Related Art

Certain devices require slow and controlled initiation and maintenance of sliding movement of one surface over another surface. It is well known that two stationary surfaces having a sliding relationship often exhibit sufficient resistance to initiation of movement that gradually increased force applied to one of the surfaces does not cause movement until a threshold force is reached, at which point a sudden sliding or shearing separation of the surfaces takes place. This sudden separation of stationary surfaces into a sliding relationship is herein referred to as "breakout" or "breakloose".

"Breakout force" refers to the force required to overcome static friction between surfaces of a syringe assembly that has been previously moved in a sliding relationship, but has been stationary ("parked" or not moved) for a short period of time (for example, milliseconds to hours). A less well known but important frictional force is "breakloose force", which refers to the force required to overcome static friction between surfaces of a syringe assembly that have not been previously moved in a sliding relationship or have been stationary for longer periods of time, often with chemical or material bonding or deformation of the surfaces due to age, sterilization, temperature cycling, or other processing.

Breakout and breakloose forces are particularly troublesome in liquid dispensing devices, such as syringes, used to deliver small, accurately measured quantities of a liquid by smooth incremental line to line advancement of one surface over a second surface. The problem is also encountered in devices using stopcocks, such as burets, pipets, addition funnels, and the like where careful dropwise control of flow is desired.

The problems of excessive breakout and breakloose forces are related to friction. Friction is generally defined as the resisting force that arises when a surface of one substance slides, or tends to slide, over an adjoining surface of itself or another substance. Between surfaces of solids in contact, there may be two kinds of friction: (1) the resistance opposing the force required to start to move one surface over another, conventionally known as static friction, and (2) the resistance opposing the force required to move one surface over another at a variable, fixed, or predetermined speed, conventionally known as kinetic friction.

The force required to overcome static friction and induce breakout or breakloose is referred to as the "breakout force" or "breakloose force", respectively, and the force required to maintain steady slide of one surface over another after breakout or breakloose is referred to as the "sustaining force". Two main factors, sticktion and inertia, contribute to static friction and thus to the breakout or breakloose force. The term "stick" or "sticktion" as used herein denotes the tendency of two surfaces in stationary contact to develop a degree of adherence to each other. The term "inertia" is conventionally defined as the indisposition to motion which must be overcome to set a mass in motion. In the context of the present invention, inertia is understood to denote that component of the breakout or breakloose force which does not involve adherence.

Breakout or breakloose forces, in particular the degree of stick, vary according to the composition of the surfaces. In general, materials having elasticity show greater stick than non-elastic materials. The length of time that surfaces have been in stationary contact with each other also influences breakout and/or breakloose forces. In the syringe art, the term "parking" denotes storage time, shelf time, or the interval between filling and discharge. Parking time generally increases breakout or breakloose force, particularly if the syringe has been refrigerated or heated during parking.

A conventional approach to overcoming breakout or breakloose has been application of a lubricant to a surface to surface interface. Common lubricants used are hydrocarbon oils, such as mineral oils, peanut oil, vegetable oils, and the like. Such products have the disadvantage of being soluble in a variety of fluids, such as vehicles commonly used to dispense medicaments. In addition, these lubricants are subject to air oxidation resulting in viscosity changes and objectionable color development. Further, they are particularly likely to migrate from the surface to surface interface. Such lubricant migration is generally thought to be responsible for the increase in breakout or breakloose force with time in parking.

In light of the above, silicone oil is typically coated in the syringe to achieve the benefits discussed above. The silicone coating can be deposited on the inner walls of the barrel by spraying, dipping or another suitable application technique. After application of the silicone coating, it is common to treat the silicone coating so prevent interaction thereof with components of the drug or medicament contained within the syringe barrel. As an example, for a silicone coating that comprises an emulsion of poly-(dimethylsiloxane), it is common for the coating to be annealed to form so-called "baked silicone", in order to make the coating less reactant with the drug/medicament contained in the syringe barrel.

While silicone oil, as well as various hydrocarbon oils, provide sufficient lubrication within the syringe, it is recognized that these oils may not be well detected in various instances. As one example, silicone oil may not be well detected when there is a low layer thickness (as with baked silicone), which may make it difficult to ascertain layer homogeneity and determine if any holes exist in the silicone oil or hydrocarbon oil coating applied on/to a glass syringe barred (e.g., a Neopak barrel). As another example, silicone oil may not be well detected when it is desired to conduct a rapid detection of the silicone oil, or hydrocarbon oil, in a syringe barrel, with 4, 5, 6, 7, or even 8 lines of measurement along the syringe diameter. As still another example, detection of X-Si particles in solution versus others type of particles during drug-syringe interaction studies may be difficult when using conventional silicone oil. Finally, silicone oil may not be well detected at the location of the glass-stopper interface.

Thus, there is needed an improved oil for use in medical devices including, but not limited to, syringes that permits for better testing, property determination, and/or quality control based on an improved contrast using one or more colorization compounds as detailed herein. In another instance, there is a need to improve the detection/observation of the silicone oil, or hydrocarbon oil, for faster analysis and comprehension of the behavior of a silicone oil, or hydrocarbon oil, with syringes, medical device stoppers, AGF tests, etc.

### SUMMARY OF THE INVENTION

The invention relates, in general, to a colorized oil comprising a combination of at least one oil (e.g., one or more silicone oils and/or one or more hydrocarbon oils) in combination with at least one colorization compound selected from one or more fluorescent compositions (e.g., one or more acrylic fluorescent compositions and/or one or more ultraviolet dyes). In one embodiment, the present invention relates to a colorized oil comprising at least one oil selected from one or more silicone oils, one or more hydrocarbon oils, or a combination of any two or more thereof, where the at least one oil has viscosity in the range of about 500 centipoise to about 1500 centipoise, in combination with at least one colorization compound selected from one or more fluorescent acrylic compositions, one or more ultraviolet dyes, or a combination of any two or more thereof, where the at least one colorization compound fluoresces at a wavelength of about 100 nm to about 400 nm.

According to one aspect, the present invention is directed to a colorized oil comprising at least one oil selected from one or more silicone oils, one or more hydrocarbon oils, or a combination of any two or more thereof, the at least one oil having a viscosity in the range of about 500 centipoise to about 1500 centipoise; and at least one colorization compound selected from one or more fluorescent acrylic compositions, one or more ultraviolet dyes, or a combination of any two or more thereof, wherein the at least one colorization compound fluoresces at a wavelength of about 100 nm to about 400 nm.

In another embodiment, the present invention is directed to a colorized silicone oil comprising at least one silicone oil, the at least one silicone oil having a viscosity in the range of about 500 centipoise to about 1500 centipoise; and at least one colorization compound selected from one or more fluorescent acrylic compositions, one or more ultraviolet dyes, or a combination of any two or more thereof, wherein the at least one colorization compound fluoresces at a wavelength of about 100 nm to about 400 nm.

In still another embodiment, the present invention is directed to a method of calibration and detection of colorized silicone oil in syringe barrels, comprising the steps of: (a) providing a syringe barrel which has an inner surface coated with a colorized silicone oil; (b) obtaining an image with an imaging system of the inner surface of the syringe barrel coated with the colorized silicone oil, wherein the imaging system is capable of detecting colorized silicone oil; and (c) determining from the image obtained from step (b) how the colorized silicone oil is interacting with at least one internal feature present in the syringe barrel.

In another aspect, the present invention is directed to a syringe comprising at least one oil composition according to any of the embodiments disclosed herein.

Clause 1: A colorized oil comprising: at least one oil selected from one or more silicone oils, one or more hydrocarbon oils, or a combination of any two or more thereof, the at least one oil having a viscosity in the range of about 500 centipoise to about 1500 centipoise; and at least one colorization compound selected from one or more fluorescent acrylic compositions, one or more ultraviolet dyes, or a combination of any two or more thereof, wherein the at least one colorization compound fluoresces at a wavelength of about 100 nm to about 400 nm.

Clause 2: The colorized oil of clause 1, wherein the at least one oil is selected from at least one silicone oil.

Clause 3: The colorized oil of any of clause 1 or 2, wherein the at least one oil is selected from at least one silicone oil having a viscosity of about 1000 centipoise.

Clause 4: The colorized oil of any of clauses 1-3, wherein the colorized oil comprises from about 0.1 percent by volume to about 3 percent by volume of the at least one colorization compound per 100 percent by volume of the colorized oil.

Clause 5: The colorized oil of any of clauses 1-4, wherein the colorized oil comprises from about 0.25 percent by volume to about 2.5 percent by volume of the at least one colorization compound per 100 percent by volume of the colorized oil.

Clause 6: The colorized oil of any of clauses 1-5, wherein the colorized oil comprises from about 0.5 percent by volume to about 2 percent by volume of the at least one colorization compound per 100 percent by volume of the colorized oil.

Clause 7: The colorized oil of any of clauses 1-6, wherein the at least one colorization compound is a fluorescent acrylic composition that fluoresces at a wavelength of about 315 to about 400 nm.

Clause 8: The colorized oil of any of clauses 1-7, wherein the at least one colorization compound is a fluorescent acrylic composition that fluoresces at a wavelength of about 365 nm.

Clause 9: A colorized silicone oil comprising: at least one silicone oil, the at least one silicone oil having a viscosity in the range of about 500 centipoise to about 1500 centipoise; and at least one colorization compound selected from one or more fluorescent acrylic compositions, one or more ultraviolet dyes, or a combination of any two or more thereof, wherein the at least one colorization compound fluoresces at a wavelength of about 100 nm to about 400 nm.

Clause 10: The colorized silicone oil of clause 9, wherein the at least one silicone oil has a viscosity of about 1000 centipoise.

Clause 11: The colorized silicone oil of any of clause 9 or 10, wherein the colorized silicone oil comprises from about 0.1 percent by volume to about 3 percent by volume of the at least one colorization compound per 100 percent by volume of the colorized silicone oil.

Clause 12: The colorized silicone oil of any of clauses 9-11, wherein the colorized silicone oil comprises from about 0.25 percent by volume to about 2.5 percent by volume of the at least one colorization compound per 100 percent by volume of the colorized silicone oil.

Clause 13: The colorized silicone oil of any of clauses 9-12, wherein the colorized silicone oil comprises from about 0.5 percent by volume to about 2 percent by volume of the at least one colorization compound per 100 percent by volume of the colorized silicone oil.

Clause 14: The colorized silicone oil of any of clauses 9-13, wherein the at least one colorization compound is a fluorescent acrylic composition that fluoresces at a wavelength of about 315 to about 400 nm.

Claus 15: The colorized silicone oil of any of clauses 9-14, wherein the at least one colorization compound is a fluorescent acrylic composition that fluoresces at a wavelength of about 365 nm.

Clause 16: A method of calibration and detection of colorized silicone oil in syringe barrels, comprising the steps of: (a) providing a syringe barrel which has an inner surface coated with a colorized silicone oil; (b) obtaining an image with an imaging system of the inner surface of the syringe barrel coated with the colorized silicone oil, wherein the imaging system is capable of detecting colorized silicone oil; and (c) determining from the image obtained from Step (b) how the colorized silicone oil is interacting with at least one internal feature present in the syringe barrel.

Clause 17: The method of clause 16, wherein the colorized silicone oil comprises: at least one silicone oil, the at least one silicone oil having a viscosity in the range of about 500 centipoise to about 1500 centipoise; and at least one colorization compound selected from one or more fluorescent acrylic compositions, one or more ultraviolet dyes, or a combination of any two or more thereof, wherein the at least one colorization compound fluoresces at a wavelength of about 100 nm to about 400 nm.

Clause 18: The method of any of clause 16 or 17, wherein the at least one colorized silicone oil has a viscosity of about 1000 centipoise.

Clause 19: The method of any of clauses 16-18, wherein the colorized silicone oil comprises from about 0.1 percent by volume to about 3 percent by volume of the at least one colorization compound per 100 percent by volume of the colorized silicone oil.

Clause 20: The method of any of clauses 16-19, wherein the colorized silicone oil comprises from about 0.25 percent by volume to about 2.5 percent by volume of the at least one colorization compound per 100 percent by volume of the colorized silicone oil.

Clause 21: The method of any of clauses 16-20, wherein the colorized silicone oil comprises from about 0.5 percent by volume to about 2 percent by volume of the at least one colorization compound per 100 percent by volume of the colorized silicone oil.

Clause 22: The method of any of clauses 16-21, wherein the at least one colorization compound is a fluorescent acrylic composition that fluoresces at a wavelength of about 315 to about 400 nm.

Clause 23: The method of any of clauses 16-22, wherein the at least one colorization compound is a fluorescent acrylic composition that fluoresces at a wavelength of about 365 nm.

### BRIEF DESCRIPTION OF THE DRAWINGS

Figure 1 is perspective view of an exemplary syringe with which embodiments of the disclosure may be implemented;
Figure 2 is a perspective view of the syringe of Figure 1 showing a needle shield covering a distal end of the syringe ;
Figure 3 is a cross-sectional side view of the barrel of the syringe of Figure 1, showing a colorized oil applied to an inner surface thererof, according to an embodiment of the present invention;
Figure 4 is a perspective view of a stopper useable with the syringe of Figure 1, according to a non-limiting embodiment;
Figure 5 is a perspective view of a stopper useable with the syringe of Figure 1, according to another non-limiting embodiment;
Figure 6 is a perspective view of a stopper useable with the syringe of Figure 1, according to another non-limiting embodiment;
Figure 7 is a perspective view of a stopper useable with the syringe of Figure 1, according to another non-limiting embodiment;
Figure 8 is a perspective view of a stopper useable with the syringe of Figure 1, according to another non-limiting embodiment; and
Figure 9 is a perspective view of a stopper useable with the syringe of Figure 1, according to another non-limiting embodiment.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof, shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

As noted above, aspects of the disclosure relate, in general, to a colorized oil comprising a combination of at least one oil (e.g., one or more silicone oils and/or one or more hydrocarbon oils) in combination with at least one colorization compound selected from one or more fluorescent compositions (e.g., one or more acrylic fluorescent compositions and/or one or more ultraviolet dyes). In one embodiment, the present invention relates to a colorized oil comprising at least one oil selected from one or more silicone oils, one or more hydrocarbon oils, or a combination of any two or more thereof, where the at least one oil has viscosity in the range of about 500 centipoise to about 1500 centipoise, in combination with at least one colorization compound selected from one or more fluorescent acrylic compositions, one or more ultraviolet dyes, or a combination of any two or more thereof, where the at least one colorization compound fluoresces at a wavelength of about 100 nm to about 400 nm.

Initially, an exemplary syringe will be described where at least one portion of the syringe, for example the syringe barrel, is formed from a suitable glass or plastic material in connection with the colorized oil of the present invention. However, it should be noted that the colorized oil of the present invention is not limited to just the syringe embodiment, or embodiments, described below, but rather, can be broadly applied to any syringe, or other medical device, that needs to undergo one or more visual tests to determine the behavior of one or more materials in a syringe and/or on a syringe stopper so as to yield better visual observation results.

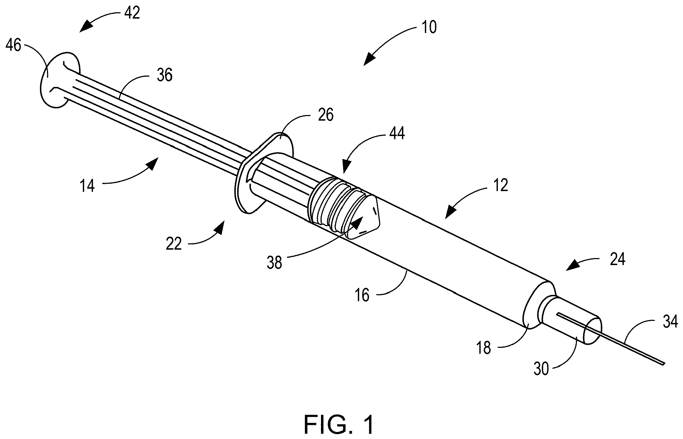

Referring to FIGS. 1-3, shown is a non-limiting embodiment of a syringe 10 with which aspects or embodiments of the disclosure may be implemented. According to some aspects of the disclosure, the syringe 10 may be provided as a prefilled syringe, which provides the convenience of rapidly delivering the liquid therein to a patient without the need to first aspirate the medication from another container and meter its volume.

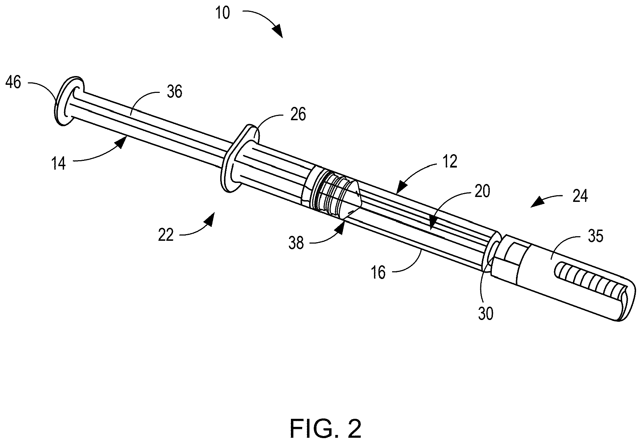

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed from a suitable glass or plastic material and includes a generally cylindrical outer wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a hub 30 which narrows with respect to the cylindrical outer wall 16 and extends out distally therefrom. The hub portion 30 is formed as a partially hollow member that defines a channel therethrough in fluid communication with the chamber 20. A needle 34 is attached to the hub portion 30 within the channel, such as by being glued or otherwise secured to the hub portion 30. According to some aspects of the disclosure, syringe 10 may further include a cover 35 that couples to the hub portion 30 of syringe barrel 12 to protect the needle 34.

The plunger assembly 14 of syringe 10 is formed of an elongate plunger rod 36 (more generally "plunger 36," as used hereafter) and a plunger head or stopper 38. The plunger 36 may include a main body 40 extending between a plunger proximal end 42 and a plunger distal end 44. A thumb press 46 is positioned at the plunger proximal end 42 that may be engaged by a thumb (or other finger) of the user to apply a distally directed force to the plunger assembly 14 to move the plunger 36 with respect to the syringe barrel 12. The plunger distal end 44 is configured to mate with the stopper 38. In other embodiments, the plunger distal end 44 may be a threaded end that mates with the stopper 38.

The stopper 38 of plunger assembly 14 is positioned at the plunger distal end 44 so as to be movable along with the plunger 36 within the chamber 20 of syringe barrel 12. That is, as a distally-directed force is applied to the plunger rod 36 (such as to thumb press 46), both the plunger rod 36 and the stopper 38 are caused to move distally through syringe barrel 12, with the stopper 38 configured to maintain a seal with an inner surface of the syringe barrel 12 as it is moved therethrough.

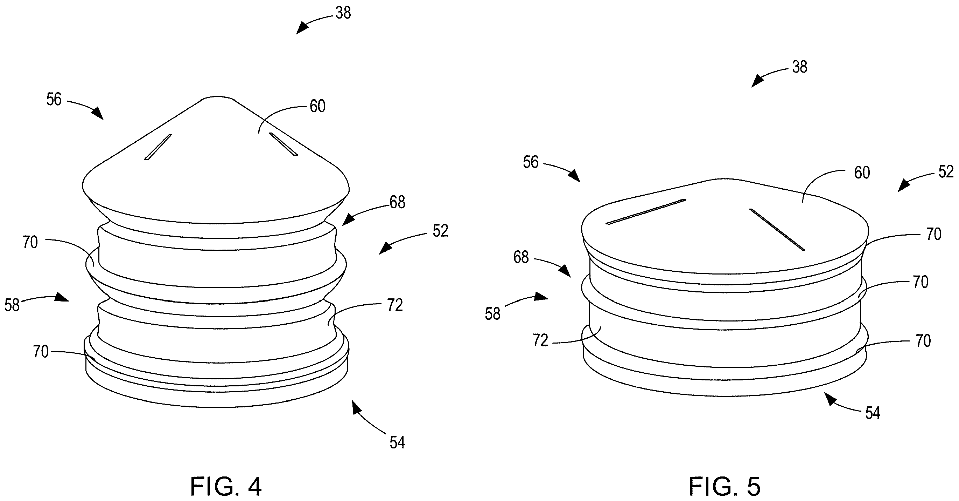

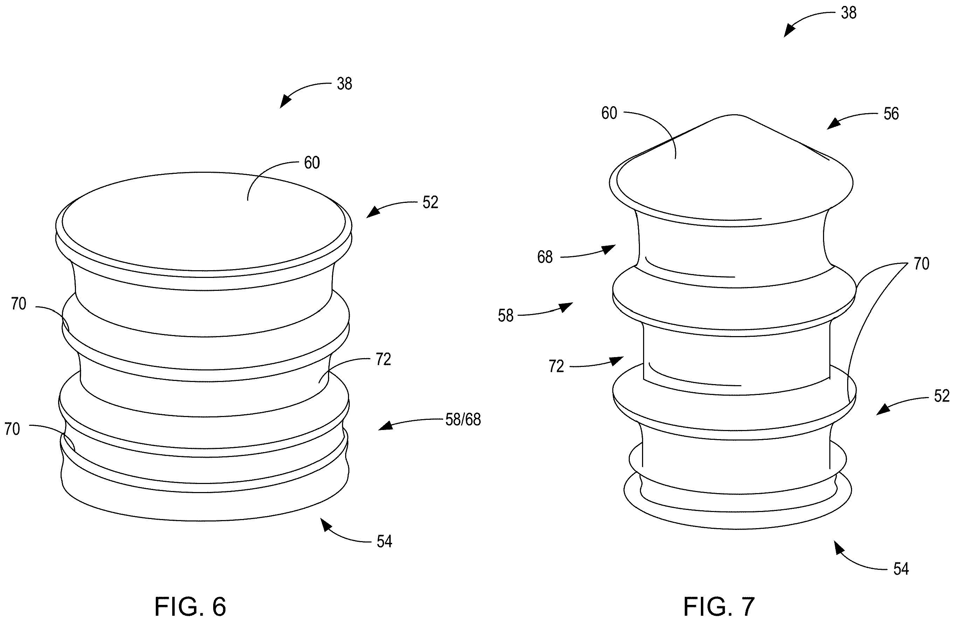

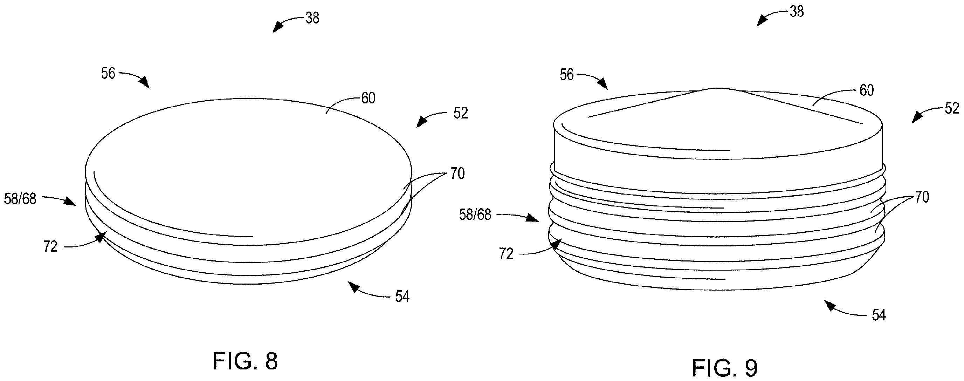

Referring now to FIGS. 4-9, and with continued reference to FIGS. 1-3, the structure of various stoppers 38 that may be included in syringe 10 is shown in greater detail, according to non-limiting aspects or embodiments of the disclosure. Each stopper 38 is defined by a main body 52 that includes a proximal end 54 and a distal end 56. The proximal end 54 may be configured as an open proximal end that engages with the distal end of plunger 36, while the distal end 56 is configured as a closed distal end configured to engage with the barrel 12 of syringe 10. The closed distal end 56 of the main body 52 includes a generally cylindrical portion 58 and a roof portion 60 that extends distally from cylindrical portion 58. In some embodiments, the roof portion 60 is configured as a conical portion (FIGS. 4, 7 and 9) that extends distally from cylindrical portion 58 to a tip to provide the closed distal end 56. In other embodiments, the roof portion 60 is formed as a flat surface (FIGS. 6 and 8) or domed surface (FIG. 5), as other non-limiting examples.

As known in the art, the main body 52 of the stopper 38 is partially hollow and is sized and configured to receive the distal end 44 of plunger 36 therein. The main body 52 of the stopper 38 defines an inner cavity (not shown) that, in some embodiments, may include a threaded inner surface that is configured to receive and mate with a threaded distal end 44of plunger 36. In other embodiments, the inner cavity may define an inner contoured surface having a notch therein that is configured to receive a flanged extension member of plunger 36.

The outer surface 68 of the cylindrical portion 58 of the main body 52 includes one or more ribs thereon that extend circumferentially around the entire outer surface 68 of the cylindrical portion 58. In each of the illustrated embodiments, a plurality of ribs 70 are included on the stopper 38, but it is recognized that other embodiments could include only a single rib 70. The ribs 70 may be formed integrally with the main body 52, such as via a molding process. The plurality of ribs 70 may include two ribs 70, with a first or distal rib positioned toward the distal end 56 of the main body 52 and a second or proximal rib positioned toward the proximal end 54 of the main body 52, or more include three or more ribs 70 spaced apart along the cylindrical portion 58 - with an inter-rib region 72 provided between each adjacent pair of ribs. The inter-rib region 74 of cylindrical portion 58 may have a generally flat outer surface 68 or a curved outer surface 68.

The stopper 38 may be made from a material that is different from the material of the plunger 36 and that is capable of forming a tight seal with the syringe barrel 12 as it is advanced therethrough. In some embodiments, the stopper 38 is formed of an elastomeric material or rubber material such as butyl, styrene butadiene, isoprene, as non-limiting examples. In other embodiments, the stopper 38 may instead be formed of a polymeric material, such as cross-linked or thermoplastic elastomers, as non-limiting examples.

According to aspects of the disclosure, the stopper 38 may further include an outer laminate or coating 75 on at least portions thereof, such as on a sealant or gliding surface thereof - i.e., on the contact surface 76 of first rib 70 and second rib 72 - and/or on roof portion 60. The laminate/coating 75 may be configured to reduce friction between the stopper 38 and barrel 12 when the stopper 38 is moved within the syringe 10. According to aspects of the disclosure, the laminate/coating 75 may be formed of one or more of ultra-high-molecular-weight polyethylene (UHMWPE), liquid silicone rubber (LSR), polydicyclopentadiene (PDCPD), polymethyl urea (PMU), polyvinylidene difluoride (PVDF), polyvinyl, a polysulfone film, or a fluoropolymer, as non-limiting examples. In some embodiments, it may be desirable for the laminate/coating 75 to be formed of a material that is free or substantially free of per- and poly- fluoroalkyl substances (PFAS).

In one embodiment, stopper 38 is desirably manufactured from any suitable polymeric material with a Young's modulus (also known as the tensile modulus, elastic modulus or traction modulus) in a range of about 4 MPa to about 9 MPa, or from about 4.25 MPa to about 8.75 MPa, or from about 4.5 MPa to about 8.5 MPa, or from about 4.75 MPa to about 8.25 MPa, or from about 5 MPa to about 8 MPa, or from about 5.25 MPa to about 7.75 MPa, or from about 5.5 MPa to about 7.5 MPa, or from about 5.75 MPa to about 7.25 MPa, or from about 6 MPa to about 7 MPa, or from about 6.25 MPa to about 6.75 MPa, or even about 6.5 MPa. In another embodiment, stopper 38 is desirably manufactured from any suitable polymeric material with a Young's modulus (also known as the tensile modulus, elastic modulus or traction modulus) in a range of about 5 MPa to about 8 MPa, or from about 5.1 MPa to about 7.9 MPa, or from about 5.2 MPa to about 7.8 MPa, or from about 5.3 MPa to about 7.7 MPa, or from about 5.4 MPa to about 7.6 MPa, or from about 5.5 MPa to about 7.5 MPa, or from about 5.6 MPa to about 7.4 MPa, or from about 5.7 MPa to about 7.3 MPa, or from about 5.8 MPa to about 7.2 MPa, or from about 5.9 MPa to about 7.1 MPa, or from about 6 MPa to about 7 MPa, or from about 6.1 MPa to about 6.9 MPa, or from about 6.2 MPa to about 6.8 MPa, or from about 6.3 MPa to about 6.7 MPa, or from about 6.4 MPa to about 6.6 MPa, or even about 6.5 MPa. Here, as well as elsewhere in the specification and claims, individual numerical values can be combined to form additional, or even new/non-disclosed, numerical ranges.

In one embodiment, the desired Shore A hardness of the afore-mentioned suitable polymer materials for stopper 38 of any of the syringes that can be used in conjunction with the present invention is between about 45 and about 85, or from about 50 to about 80, or from about 55 to about 75, or from about 60 to about 70, or even about 65. In another embodiment, stopper 38 is desirably manufactured from any suitable polymeric material with a Shore A hardness of about 50 to about 80, or from about 52 to about 78, or from about 54 to about 76, or from about 56 to about 74, or from about 58 to about 72, or from about 60 to about 70, or from about 62 to about 68, or from about 64 to about 66, or even about 65. Here, as well as elsewhere in the specification and claims, individual numerical values can be combined to form additional, or even new/non-disclosed, numerical ranges.

Suitable stopper polymeric materials include, but are not limited to, one or more polyolefins (e.g., PE, PP, and their copolymers), one or more polyamides (e.g., nylons), one or more polyesters (e.g., PET), one or more polystyrenes, one or more polyurethanes, one or more polycarbonates, one or more acrylonitrile-butadiene-styrenes, one or more fluoropolymers, one or more polyacrylates, one or more elastomeric rubbers, or blends of any two or more thereof that meet at least one of the Young's modulus and/or Shore A properties defined above. In another embodiment, suitable stopper materials include, but are not limited to, polymeric materials selected from one or more polyisoprenes (including, but not limited to, natural rubber), one or more polyisobutylenes, one or more synthetic polyisoprenes, one or more polybutadienes (commonly referred to collectively as butadiene rubbers), one or more chloroprene rubbers (e.g., polychloroprene, neoprene, etc.), one or more butyl rubbers (i.e., copolymers of isobutene and isoprene), one or more halogenated butyl rubbers (e.g., chloro butyl rubber or bromo butyl rubber), one or more styrene-butadiene rubbers (i.e., copolymers of styrene and butadiene), one or more nitrile rubbers (i.e., copolymers of butadiene and acrylonitrile), one or more hydrogenated nitrile rubbers, natural rubber, one or more ethylene propylene rubbers, one or more ethylene propylene diene rubbers, or blends of any two or more thereof. In another embodiment, suitable stopper polymeric materials include, but are not limited to, one or more butadiene rubbers, one or more halogenated butyl rubbers (e.g., chloro butyl rubber or bromo butyl rubber), or blends of any two or more thereof.

In another embodiment, stopper 38 is desirably manufactured from any suitable polymeric material with either a Young's modulus and/or a Shore A hardness in one of the ranges noted above in combination with a high Mooney viscosity. A high Mooney rubber is defined as rubber having Mooney viscosity greater than about 40, or greater than about 45, or greater than about 50, or greater than about 55, or greater than about 60, or greater than about 65, or even greater than about 70. Here, as well as elsewhere in the specification and claims, individual numerical values can be combined to form additional, or even new/non-disclosed, numerical ranges.

Suitable high Mooney viscosity rubbers include, but are not limited to, one or more polybutadiene rubbers, one or more polyisobutylene, one or more polyisoprene rubbers, natural rubber, one or more ethylene propylene rubbers, one or more ethylene propylene diene rubbers, one or more styrene-butadiene rubbers, or blends of any two or more thereof.

In one embodiment, stopper 38 is manufactured from a polymeric material with a significantly lower coefficient of friction and contact area/pressure relative to a conventional rubber stopper. Accordingly, in this embodiment, stopper 38 can be used with a syringe 10 without silicone oil or other lubricants. In addition, syringe barrel 12 may be made of the same material, a similar material, or even a completely different material from that used to form stopper 38.

In one embodiment, due to the polymeric material that is used to form the stopper of any of the syringes that can be used in conjunction with the present invention (or any of the disclosed alternative stopper designs incorporated herein by reference), suitable sealing is accomplished by the contact pressure achieved by any one or more of a stopper in accordance with the syringes that can be used in conjunction with the present invention where such a stopper is formed from any one or more of the polymeric materials having one or more of the properties disclosed herein. That is, the suitable sealing properties of the stoppers of any of the syringes that can be used in conjunction with the present invention are achieved due to one or more of stopper geometry, the Young's modulus of the polymeric material used to form such a stopper, the indentation modulus of the laminate sealing surface, and/or the roughness of both the surface of the stopper and/or the inner surface of the barrel (or other container) that the stopper is designed to seal.

In one embodiment, the surface roughness of the glass, polymer, metal or another type of material that forms the surface to be sealed (such as a vial, syringe, or other type of container) by the stopper of any of the syringes that can be used in conjunction with the present invention is independently in a range of about 0.0001 microns to about 6 microns, or from about 0.0002 microns to about 5.9 microns, or from about 0.0003 microns to about 5.8 microns, or from about 0.0004 microns to about 5.7 microns, or from about 0.0005 microns to about 5.6 microns, or from about 0.0006 microns to about 5.5 microns, or from about 0.0007 microns to about 5.4 microns, or from about 0.0008 microns to about 5.3 microns, or from about 0.0009 microns to about 5.2 microns, or from about 0.001 microns to about 5.1 microns, or from about 0.0011 microns to about 5 microns, or from about 0.0012 microns to about 4.9 microns, or from about 0.0013 microns to about 4.8 microns, or from about 0.0014 microns to about 4.7 microns, or from about 0.0015 microns to about 4.6 microns, or from about 0.0016 microns to about 4.5 microns, or from about 0.0017 microns to about 4.4 microns, or from about 0.0018 microns to about 4.3 microns, or from about 0.0019 microns to about 4.2 microns, or from about 0.002 microns to about 4.1 microns, or from about 0.0021 microns to about 4 microns, or from about 0.0022 microns to about 3.9 microns, or from about 0.0023 microns to about 3.8 microns, or from about 0.0024 microns to about 3.7 microns, or from about 0.0025 microns to about 3.6 microns, or from about 0.0026 microns to about 3.5 microns, or from about 0.0027 microns to about 3.4 microns, or from about 0.0028 microns to about 3.3 microns, or from about 0.0029 microns to about 3.2 microns, or from about 0.003 microns to about 3.1 microns, or from about 0.0031 microns to about 3 microns, or from about 0.0032 microns to about 2.9 microns, or from about 0.0033 microns to about 2.8 microns, or from about 0.0034 microns to about 2.7 microns, or from about 0.0035 microns to about 2.6 microns, or from about 0.0036 microns to about 2.5 microns, or from about 0.0037 microns to about 2.4 microns, or from about 0.0038 microns to about 2.3 microns, or from about 0.0039 microns to about 2.2 microns, or from about 0.004 microns to about 2.1 microns, or from about 0.0041 microns to about 2 microns, or from about 0.0042 microns to about 1.9 microns, or from about 0.0043 microns to about 1.8 microns, or from about 0.0044 microns to about 1.7 microns, or from about 0.0045 microns to about 1.6 microns, or from about 0.0046 microns to about 1.5 microns, or from about 0.0047 microns to about 1.4 microns, or from about 0.0048 microns to about 1.3 microns, or from about 0.0049 microns to about 1.2 microns, or from about 0.005 microns to about 1.1 microns, or from about 0.0051 microns to about 1 micron, or from about 0.0052 microns to about 0.9 microns, or from about 0.0053 microns to about 0.8 microns, or from about 0.0054 microns to about 0.7 microns, or from about 0.0055 microns to about 0.6 microns, or from about 0.0056 microns to about 0.5 microns, or from about 0.0057 microns to about 0.4 microns, or from about 0.0058 microns to about 0.3 microns, or from about 0.0059 microns to about 0.2 microns, or from about 0.006 microns to about 0.1 microns, or from about 0.0065 microns to about 0.095 microns, or from about 0.007 microns to about 0.09 microns, or from about 0.0075 microns to about 0.085 microns, or from about 0.008 microns to about 0.08 microns, or from about 0.0085 microns to about 0.075 microns, or from about 0.009 microns to about 0.07 microns, or from about 0.0095 microns to about 0.065 microns, or from about 0.01 microns to about 0.06 microns, or from about 0.015 microns to about 0.055 microns, or from about 0.02 microns to about 0.05 microns, or from about 0.025 microns to about 0.045 microns, or from about 0.03 microns to about 0.04 microns, or even 0.035 microns as measured at 50X via optical interferometry. Here, as well as elsewhere in the specification and claims, individual numerical values can be combined to form additional, or even new/non-disclosed, numerical ranges.

In one embodiment, the surface roughness of the polymeric material that is used to form the stopper of any of the syringes that can be used in conjunction with the present invention is independently in a range of about 0.0001 microns to about 6 microns, or from about 0.0002 microns to about 5.9 microns, or from about 0.0003 microns to about 5.8 microns, or from about 0.0004 microns to about 5.7 microns, or from about 0.0005 microns to about 5.6 microns, or from about 0.0006 microns to about 5.5 microns, or from about 0.0007 microns to about 5.4 microns, or from about 0.0008 microns to about 5.3 microns, or from about 0.0009 microns to about 5.2 microns, or from about 0.001 microns to about 5.1 microns, or from about 0.0011 microns to about 5 microns, or from about 0.0012 microns to about 4.9 microns, or from about 0.0013 microns to about 4.8 microns, or from about 0.0014 microns to about 4.7 microns, or from about 0.0015 microns to about 4.6 microns, or from about 0.0016 microns to about 4.5 microns, or from about 0.0017 microns to about 4.4 microns, or from about 0.0018 microns to about 4.3 microns, or from about 0.0019 microns to about 4.2 microns, or from about 0.002 microns to about 4.1 microns, or from about 0.0021 microns to about 4 microns, or from about 0.0022 microns to about 3.9 microns, or from about 0.0023 microns to about 3.8 microns, or from about 0.0024 microns to about 3.7 microns, or from about 0.0025 microns to about 3.6 microns, or from about 0.0026 microns to about 3.5 microns, or from about 0.0027 microns to about 3.4 microns, or from about 0.0028 microns to about 3.3 microns, or from about 0.0029 microns to about 3.2 microns, or from about 0.003 microns to about 3.1 microns, or from about 0.0031 microns to about 3 microns, or from about 0.0032 microns to about 2.9 microns, or from about 0.0033 microns to about 2.8 microns, or from about 0.0034 microns to about 2.7 microns, or from about 0.0035 microns to about 2.6 microns, or from about 0.0036 microns to about 2.5 microns, or from about 0.0037 microns to about 2.4 microns, or from about 0.0038 microns to about 2.3 microns, or from about 0.0039 microns to about 2.2 microns, or from about 0.004 microns to about 2.1 microns, or from about 0.0041 microns to about 2 microns, or from about 0.0042 microns to about 1.9 microns, or from about 0.0043 microns to about 1.8 microns, or from about 0.0044 microns to about 1.7 microns, or from about 0.0045 microns to about 1.6 microns, or from about 0.0046 microns to about 1.5 microns, or from about 0.0047 microns to about 1.4 microns, or from about 0.0048 microns to about 1.3 microns, or from about 0.0049 microns to about 1.2 microns, or from about 0.005 microns to about 1.1 microns, or from about 0.0051 microns to about 1 micron, or from about 0.0052 microns to about 0.9 microns, or from about 0.0053 microns to about 0.8 microns, or from about 0.0054 microns to about 0.7 microns, or from about 0.0055 microns to about 0.6 microns, or from about 0.0056 microns to about 0.5 microns, or from about 0.0057 microns to about 0.4 microns, or from about 0.0058 microns to about 0.3 microns, or from about 0.0059 microns to about 0.2 microns, or from about 0.006 microns to about 0.1 microns, or from about 0.0065 microns to about 0.095 microns, or from about 0.007 microns to about 0.09 microns, or from about 0.0075 microns to about 0.085 microns, or from about 0.008 microns to about 0.08 microns, or from about 0.0085 microns to about 0.075 microns, or from about 0.009 microns to about 0.07 microns, or from about 0.0095 microns to about 0.065 microns, or from about 0.01 microns to about 0.06 microns, or from about 0.015 microns to about 0.055 microns, or from about 0.02 microns to about 0.05 microns, or from about 0.025 microns to about 0.045 microns, or from about 0.03 microns to about 0.04 microns, or even 0.035 microns as measured at 50X via optical interferometry. Here, as well as elsewhere in the specification and claims, individual numerical values can be combined to form additional, or even new/non-disclosed, numerical ranges.

In still another embodiment, if the surface to be sealed is partially, or fully, laminated the surface roughness of the laminated film, or the top most laminated film in the case where two or more films are used to provide an overall laminated coating, is independently in a range of about 0.0001 microns to about 12 microns, or from about 0.0002 microns to about 11.9 microns, or from about 0.0003 microns to about 11.8 microns, or from about 0.0004 microns to about 11.7 microns, or from about 0.0005 microns to about 11.6 microns, or from about 0.0006 microns to about 11.5 microns, or from about 0.0007 microns to about 11.4 microns, or from about 0.0008 microns to about 11.3 microns, or from about 0.0009 microns to about 11.2 microns, or from about 0.001 microns to about 11.1 microns, or from about 0.0011 microns to about 11 microns, or from about 0.0012 microns to about 10.9 microns, or from about 0.0013 microns to about 10.8 microns, or from about 0.0014 microns to about 10.7 microns, or from about 0.0015 microns to about 10.6 microns, or from about 0.0016 microns to about 10.5 microns, or from about 0.0017 microns to about 10.4 microns, or from about 0.0018 microns to about 10.3 microns, or from about 0.0019 microns to about 10.2 microns, or from about 0.002 microns to about 10.1 microns, or from about 0.0021 microns to about 10 microns, or from about 0.0022 microns to about 9.9 microns, or from about 0.0023 microns to about 9.8 microns, or from about 0.0024 microns to about 9.7 microns, or from about 0.0025 microns to about 9.6 microns, or from about 0.0026 microns to about 9.5 microns, or from about 0.0027 microns to about 9.4 microns, or from about 0.0028 microns to about 9.3 microns, or from about 0.0029 microns to about 9.2 microns, or from about 0.003 microns to about 9.1 microns, or from about 0.0031 microns to about 9 microns, or from about 0.0032 microns to about 8.9 microns, or from about 0.0033 microns to about 8.8 microns, or from about 0.0034 microns to about 8.7 microns, or from about 0.0035 microns to about 8.6 microns, or from about 0.0036 microns to about 8.5 microns, or from about 0.0037 microns to about 8.4 microns, or from about 0.0038 microns to about 8.3 microns, or from about 0.0039 microns to about 8.2 microns, or from about 0.004 microns to about 8.1 microns, or from about 0.0041 microns to about 8 microns, or from about 0.0042 microns to about 7.9 microns, or from about 0.0043 microns to about 7.8 microns, or from about 0.0044 microns to about 7.7 microns, or from about 0.0045 microns to about 7.6 microns, or from about 0.0046 microns to about 7.5 microns, or from about 0.0047 microns to about 7.4 microns, or from about 0.0048 microns to about 7.3 microns, or from about 0.0049 microns to about 7.2 microns, or from about 0.005 microns to about 7.1 microns, or from about 0.0051 microns to about 7 microns, or from about 0.0052 microns to about 6.9 microns, or from about 0.0053 microns to about 6.8 microns, or from about 0.0054 microns to about 6.7 microns, or from about 0.0055 microns to about 6.6 microns, or from about 0.0056 microns to about 6.5 microns, or from about 0.0057 microns to about 6.4 microns, or from about 0.0058 microns to about 6.3 microns, or from about 0.0059 microns to about 6.2 microns, or from about 0.006 microns to about 6.1 microns, or from about 0.0065 microns to about 6 microns, or from about 0.007 microns to about 5.9 microns, or from about 0.0075 microns to about 5.8 microns, or from about 0.008 microns to about 5.7 microns, or from about 0.0085 microns to about 5.6 microns, or from about 0.009 microns to about 5.5 microns, or from about 0.0095 microns to about 5.4 microns, or from about 0.01 microns to about 5.3 microns, or from about 0.015 microns to about 5.2 microns, or from about 0.02 microns to about 5.1 microns, or from about 0.025 microns to about 5 microns, or from about 0.03 microns to about 4.9 microns, or from about 0.035 microns to about 4.8 microns, or from about 0.04 microns to about 4.7 microns, or from about 0.045 microns to about 4.6 microns, or from about 0.05 microns to about 4.5 microns, or from about 0.055 microns to about 4.4 microns, or from about 0.06 microns to about 4.3 microns, or from about 0.065 microns to about 4.2 microns, or from about 0.07 microns to about 4.1 microns, or from about 0.075 microns to about 4 microns, or from about 0.08 microns to about 3.9 microns, or from about 0.085 microns to about 3.8 microns, or from about 0.09 microns to about 3.7 microns, or from about 0.095 microns to about 3.6 microns, or from about 0.1 microns to about 3.5 microns, or from about 0.15 microns to about 3.4 microns, or from about 0.2 microns to about 3.3 microns, or from about 0.25 microns to about 3.2 microns, or from about 0.3 microns to about 3.1 microns, or from about 0.35 microns to about 3 microns, or from about 0.4 microns to about 2.9 microns, or from about 0.45 microns to about 2.8 microns, or from about 0.5 microns to about 2.7 microns, or from about 0.55 microns to about 2.6 microns, or from about 0.6 microns to about 2.5 microns, or from about 0.65 microns to about 2.4 microns, or from about 0.7 microns to about 2.3 microns, or from about 0.75 microns to about 2.2 microns, or from about 0.8 microns to about 2.1 microns, or from about 0.85 microns to about 2 microns, or from about 0.9 microns to about 1.9 microns, or from about 0.95 microns to about 1.8 microns, or from about 1 micron to about 1.7 microns, or from about 1.1 microns to about 1.6 microns, or from about 1.2 microns to about 1.5 microns, or from about 1.3 microns to about 1.4 microns, or even about 1.35 microns as measured at 50X via optical interferometry. Here, as well as elsewhere in the specification and claims, individual numerical values can be combined to form additional, or even new/non-disclosed, numerical ranges.

In still another embodiment, if the stopper of any of the syringes that can be used in conjunction with the present invention is partially, or fully, laminated the surface roughness of the laminated film, or the top most laminated film in the case where two or more films are used to provide an overall laminated coating, is independently in a range of about 0.0001 microns to about 6 microns, or from about 0.0002 microns to about 5.9 microns, or from about 0.0003 microns to about 5.8 microns, or from about 0.0004 microns to about 5.7 microns, or from about 0.0005 microns to about 5.6 microns, or from about 0.0006 microns to about 5.5 microns, or from about 0.0007 microns to about 5.4 microns, or from about 0.0008 microns to about 5.3 microns, or from about 0.0009 microns to about 5.2 microns, or from about 0.001 microns to about 5.1 microns, or from about 0.0011 microns to about 5 microns, or from about 0.0012 microns to about 4.9 microns, or from about 0.0013 microns to about 4.8 microns, or from about 0.0014 microns to about 4.7 microns, or from about 0.0015 microns to about 4.6 microns, or from about 0.0016 microns to about 4.5 microns, or from about 0.0017 microns to about 4.4 microns, or from about 0.0018 microns to about 4.3 microns, or from about 0.0019 microns to about 4.2 microns, or from about 0.002 microns to about 4.1 microns, or from about 0.0021 microns to about 4 microns, or from about 0.0022 microns to about 3.9 microns, or from about 0.0023 microns to about 3.8 microns, or from about 0.0024 microns to about 3.7 microns, or from about 0.0025 microns to about 3.6 microns, or from about 0.0026 microns to about 3.5 microns, or from about 0.0027 microns to about 3.4 microns, or from about 0.0028 microns to about 3.3 microns, or from about 0.0029 microns to about 3.2 microns, or from about 0.003 microns to about 3.1 microns, or from about 0.0031 microns to about 3 microns, or from about 0.0032 microns to about 2.9 microns, or from about 0.0033 microns to about 2.8 microns, or from about 0.0034 microns to about 2.7 microns, or from about 0.0035 microns to about 2.6 microns, or from about 0.0036 microns to about 2.5 microns, or from about 0.0037 microns to about 2.4 microns, or from about 0.0038 microns to about 2.3 microns, or from about 0.0039 microns to about 2.2 microns, or from about 0.004 microns to about 2.1 microns, or from about 0.0041 microns to about 2 microns, or from about 0.0042 microns to about 1.9 microns, or from about 0.0043 microns to about 1.8 microns, or from about 0.0044 microns to about 1.7 microns, or from about 0.0045 microns to about 1.6 microns, or from about 0.0046 microns to about 1.5 microns, or from about 0.0047 microns to about 1.4 microns, or from about 0.0048 microns to about 1.3 microns, or from about 0.0049 microns to about 1.2 microns, or from about 0.005 microns to about 1.1 microns, or from about 0.0051 microns to about 1 micron, or from about 0.0052 microns to about 0.9 microns, or from about 0.0053 microns to about 0.8 microns, or from about 0.0054 microns to about 0.7 microns, or from about 0.0055 microns to about 0.6 microns, or from about 0.0056 microns to about 0.5 microns, or from about 0.0057 microns to about 0.4 microns, or from about 0.0058 microns to about 0.3 microns, or from about 0.0059 microns to about 0.2 microns, or from about 0.006 microns to about 0.1 microns, or from about 0.0065 microns to about 0.095 microns, or from about 0.007 microns to about 0.09 microns, or from about 0.0075 microns to about 0.085 microns, or from about 0.008 microns to about 0.08 microns, or from about 0.0085 microns to about 0.075 microns, or from about 0.009 microns to about 0.07 microns, or from about 0.0095 microns to about 0.065 microns, or from about 0.01 microns to about 0.06 microns, or from about 0.015 microns to about 0.055 microns, or from about 0.02 microns to about 0.05 microns, or from about 0.025 microns to about 0.045 microns, or from about 0.03 microns to about 0.04 microns, or even 0.035 microns as measured at 50X via optical interferometry. Here, as well as elsewhere in the specification and claims, individual numerical values can be combined to form additional, or even new/non-disclosed, numerical ranges.

In the embodiment where the syringe barrel is formed from glass, the compression ratio of the stopper versus the glass barrel inner diameter is from about 1 percent to about to about 10 percent, or from about 1.25 percent to about 9.75 percent, or from about 1.5 to about 9.5 percent, or from about 1.75 percent to about 9.25 percent, or from about 2 percent to about 9 percent, or from about 2.25 percent to about 8.75 percent, or from about 2.5 to about 8.5 percent, or from about 2.75 percent to about 8.25 percent, or from about 3 percent to about 8 percent, or from about 3.25 percent to about 7.75 percent, or from about 3.5 to about 7.5 percent, or from about 3.75 percent to about 7.25 percent, or from about 4 percent to about 7 percent, or from about 4.25 percent to about 6.75 percent, or from about 4.5 to about 6.5 percent, or from about 4.75 percent to about 6.25 percent, or from about 5 percent to about 6 percent, or from about 5.25 percent to about 5.75 percent, or even about 5.5 percent as determined from nominal dimensions meaning maximum outer diameter of the stopper in free state versus minimum sealing barrel inner diameter (consider in some instances that there may be a draft on the barrel inner diameter). Here, as well as elsewhere in the specification and claims, individual numerical values can be combined to form additional, or even new/non-disclosed, numerical ranges.

In one embodiment, the contact pressure generated at the sealing surface (be it glass, polymer, metal or another type of material that forms the surface to be sealed) by a stopper formed from a polymeric material in accordance with any of the syringes that can be used in conjunction with the present invention as measured within about a 0.01 mm width is more than about 90 percent of the indentation modulus, or yield strength, of a corresponding film of the polymeric material used to form such a stopper. As is known, the indentation modulus describes the elastic surface behavior of a material, in this case a polymeric material, during indentation. In another embodiment, the contact pressure generated at the sealing surface as measured within about a 0.01 mm width is more than about 91 percent, more than about 92 percent, more than about 93 percent, more than about 94 percent, more than about 95 percent, or even more than about 96 percent of the indentation modulus, or yield strength, of a corresponding film of the polymeric material used to form such a stopper. Here, as well as elsewhere in the specification and claims, individual numerical values can be combined to form additional, or even new/non-disclosed, numerical ranges. Alternatively, any of the contact pressure values noted above can be determined by comparing same to the contact pressure of a desired polymeric material at 50 percent elongation modulus.

In one embodiment, the polymeric material that is used to form a stopper of any of the syringes that can be used in conjunction with the present invention has a rubber substrate modulus in a range of about 4 MPa to about 8 MPa, or from about 4.25 MPa to about 7.75 MPa, or from about 4.5 MPa to about 7.5 MPa, or from about 4.75 MPa to about 7.25 MPa, or from about 5 MPa to about 7 MPa, or from about 5.25 MPa to about 6.75 MPa, or from about 5.5 MPa to about 6.5 MPa, or from about 5.75 MPa to about 6.25 MPa, or even about 6 MPa as measured at the limit of the stress versus strain of a molded part formed from a desired polymeric material without a laminated film thereon. Here, as well as elsewhere in the specification and claims, individual numerical values can be combined to form additional, or even new/non-disclosed, numerical ranges.

In one embodiment, any of the syringes that can be used in conjunction with the present invention use various correlations between stopper geometry, roughness, indentation modulus of the surfaces, and/or modulus of substrate materials to achieve sealing (percolation threshold) that is independent of the surfaces energies of the sealing surfaces. As such, in one embodiment, the stoppers of any of the syringes that can be used in conjunction with the present invention are partially laminated and therefore each stopper has an elastomeric body, one or more fluoropolymer layers, and one or more (or even two or more ribs) laminated with the one or more fluoropolymer layers. In another embodiment, the stoppers of any of the syringes that can be used in conj unction with the present invention are fully laminated with all of the stopper being laminated with one or more fluoropolymer layers. In one embodiment, the one or more fluoropolymer layers described herein may include a single layer of densified expanded polytetrafluoroethylene (ePTFE), or the one or more fluoropolymer layers may include a composite fluoropolymer film having a barrier layer and a porous layer, the barrier layer including densified ePTFE, polytetrafluoroethylene (PTFE), fluorinated ethylene propylene (FEP), polyethylene, polypropylene, polyvinylidene fluoride, polyvinylfluoride, perfluoropropylevinylether, a perfluoroalkoxy polymer, and copolymers and combinations thereof. In another embodiment, any high temperature material, such as a polymeric material, can be used to form the one or more lamination layers.

In another embodiment, the stoppers of the present invention are partially laminated and each stopper has a body formed from any polymeric material disclosed herein. In another embodiment, the stoppers of the present invention are partially laminated and each stopper has a body formed from any polymeric material that possesses, at a minimum, both a Young's modulus and Shore A hardness as disclosed herein, and that can be processed as described herein. In one instance, the body of any of the stoppers disclosed herein can be partially, or even fully, laminated with one or more fluoropolymer layers, and have one or more ribs (or even two or more ribs) laminated with the one or more fluoropolymer layers. In another embodiment, the stoppers of the present invention are fully laminated with all of the stopper body and ribs being laminated with one or more fluoropolymer layers. In one embodiment, the one or more fluoropolymer layers described herein may include a single layer of densified expanded polytetrafluoroethylene (ePTFE), or the one or more fluoropolymer layers may include a composite fluoropolymer film having a barrier layer and a porous layer, the barrier layer including densified ePTFE, polytetrafluoroethylene (PTFE), fluorinated ethylene propylene (FEP), polyethylene (e.g., UHMWPE), polypropylene (e.g., syndiotactic-PP), polyvinylidene fluoride, polyvinylfluoride, perfluoropropylevinylether, a perfluoroalkoxy polymer, polysulfone, and copolymers and combinations thereof. In still another embodiment, any high temperature material, such as a polymeric material, can be used to form the one or more lamination layers regardless of whether a stopper according to this embodiment is partially, or even fully, laminated. In still another embodiment, the stoppers of the present invention are laminated with one or more non-fluoropolymer materials. Suitable non-fluoropolymer materials for such lamination layers include, but are not limited to, UHMWPE, syndiotactic-polypropylene blends, and/or polysulfone.

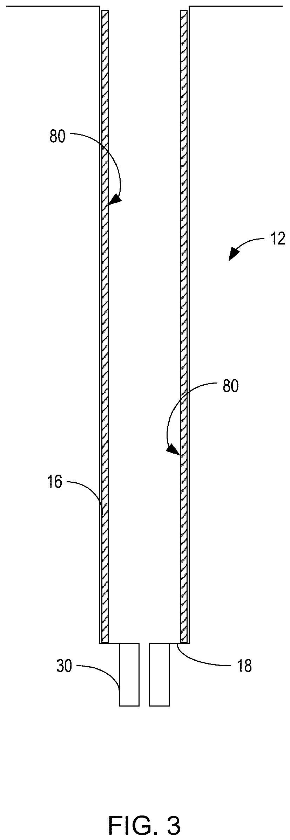

As best shown in FIG. 3, for the syringe 10 described above, a colorized oil 80 may be applied to at least one portion of the syringe, such as the syringe barrel 12. Following here below is a discussion of one type of exemplary colorized oil 80. However, it is to be understood that the following colorized oil composition is only representative and the present invention is not limited to solely the various compositions disclosed herein. Rather, any colorized oil composition within the spirt of the disclosure contained herein can be utilized in conjunction with the present invention.

In one embodiment, the colorized oil of the present invention comprises at least one oil selected from one or more silicone oils, one or more hydrocarbon oils, or a combination of any two or more thereof having a viscosity in the range of about 500 centipoise to about 1500 centipoise in combination with at least one colorization compound selected from one or more fluorescent acrylic compositions, one or more ultraviolet dyes, or a combination of any two or more thereof, where the at least one colorization compound fluoresces at a wavelength of about 100 nm to about 400 nm.

Given the above, in one embodiment the oil component of the present invention is one or more silicone oils selected from one or more polydimethylsiloxanes (PDMS) (also known as dimethicone), a mixture of polydimethylsiloxanes (PDMS) with simethicone, polymethyl hydrogen siloxane oil, amino silicone oil, phenyl methyl silicone oil, vinyl silicone oil, hydrogen silicone oil, or combinations of two or more thereof so long as the viscosity, or overall viscosity, of the one or more silicone oils is in the range of about 500 centipoise to about 1500 centipoise. In another embodiment, the oil component of the present invention can be selected from any one or more suitable food grade oils and/or any one or more medical grade oils so long as such an oil, or mixtures of oils, has a viscosity, or overall viscosity, in the range of about 500 centipoise to about 1500 centipoise. In still another embodiment, the oil component of the present invention is a combination of one or more silicone oils with one or more hydrocarbon oils so long as the viscosity, or overall viscosity, of the combined oil component is in the range of about 500 centipoise to about 1500 centipoise. In still yet another embodiment, the oil component of the present invention can be selected from any one or more of the various oils disclosed above in combination with any one or more suitable viscosity modifiers so long as such a one or more oil-viscosity modifier mixture has a viscosity, or overall viscosity, in the range of about 500 centipoise to about 1500 centipoise. It should be noted that in this embodiment, any one or more viscosity modifiers are selected so as to not interfere with the UV properties of the at least one colorization compound used in connection with the present invention. In one embodiment, suitable viscosity modifiers include, but are not limited to, any viscosity modifiers that are suitable for use in connection with food-grade and/or medical-grade applications so long as such viscosity modifiers do not interfere with the UV properties of the at least one colorization compound used in connection with the present invention. In another embodiment, a suitable medical-grade viscosity modifier can be selected from any suitable polymer-based medical-grade viscosity modifier so long as such viscosity modifiers do not interfere with the UV properties of the at least one colorization compound used in connection with the present invention.

In another embodiment, the viscosity of the oil component of the present invention is in the range of about 510 centipoise to about 1490 centipoise, or from about 520 centipoise to about 1480 centipoise, or from about 530 centipoise to about 1470 centipoise, or from about 540 centipoise to about 1460 centipoise, or from about 550 centipoise to about 1450 centipoise, or from about 560 centipoise to about 1440 centipoise, or from about 570 centipoise to about 1430 centipoise, or from about 580 centipoise to about 1420 centipoise, or from about 590 centipoise to about 1410 centipoise, or from about 600 centipoise to about 1400 centipoise, or from about 610 centipoise to about 1390 centipoise, or from about 620 centipoise to about 1380 centipoise, or from about 630 centipoise to about 1370 centipoise, or from about 640 centipoise to about 1360 centipoise, or from about 650 centipoise to about 1350 centipoise, or from about 660 centipoise to about 1340 centipoise, or from about 670 centipoise to about 1330 centipoise, or from about 680 centipoise to about 1320 centipoise, or from about 690 centipoise to about 1310 centipoise, or from about 700 centipoise to about 1300 centipoise, or from about 710 centipoise to about 1290 centipoise, or from about 720 centipoise to about 1280 centipoise, or from about 730 centipoise to about 1270 centipoise, or from about 740 centipoise to about 1260 centipoise, or from about 750 centipoise to about 1250 centipoise, or from about 760 centipoise to about 1240 centipoise, or from about 770 centipoise to about 1230 centipoise, or from about 780 centipoise to about 1220 centipoise, or from about 790 centipoise to about 1210 centipoise, or from about 800 centipoise to about 1200 centipoise, or from about 810 centipoise to about 1190 centipoise, or from about 820 centipoise to about 1180 centipoise, or from about 830 centipoise to about 1170 centipoise, or from about 840 centipoise to about 1160 centipoise, or from about 850 centipoise to about 1150 centipoise, or from about 860 centipoise to about 1140 centipoise, or from about 870 centipoise to about 1130 centipoise, or from about 880 centipoise to about 1120 centipoise, or from about 890 centipoise to about 1110 centipoise, or from about 900 centipoise to about 1100 centipoise, or from about 910 centipoise to about 1090 centipoise, or from about 920 centipoise to about 1080 centipoise, or from about 930 centipoise to about 1070 centipoise, or from about 940 centipoise to about 1060 centipoise, or from about 950 centipoise to about 1050 centipoise, or from about 960 centipoise to about 1040 centipoise, or from about 970 centipoise to about 1030 centipoise, or from about 980 centipoise to about 1020 centipoise, or from about 990 centipoise to about 1010 centipoise, or even about 1000 centipoise. Here, as well as elsewhere in the specification and claims, individual numerical values can be combined to form additional, or even new/non-disclosed, numerical ranges.

Given the above, in one embodiment the at least one colorization compound is selected from one or more fluorescent acrylic compositions (e.g., Daler Rowney System 3 acrylic and/or Golden High flow acrylic fluorescent chartreuse), one or more ultraviolet dyes, or a combination of any two or more thereof so long as the at least one colorization compound fluoresces at a wavelength of about 100 nm to about 400 nm.

In another embodiment, the at least one colorization compound of the present invention fluoresces at a wavelength of about 110 nm to about 390 nm, or from about 120 nm to about 380 nm, or from about 130 nm to about 370 nm, or from about 140 nm to about 360 nm, or from about 150 nm to about 350 nm, or from about 160 nm to about 340 nm, or from about 170 nm to about 330 nm, or from about 180 nm to about 320 nm, or from about 190 nm to about 310 nm, or from about 200 nm to about 300 nm, or from about 210 nm to about 290 nm, or from about 220 nm to about 280 nm, or from about 230 nm to about 270 nm, or from about 240 nm to about 260 nm, or even about 250 nm. Here, as well as elsewhere in the specification and claims, individual numerical values can be combined to form additional, or even new/non-disclosed, numerical ranges.

Given the above, in one embodiment the amount of the at least one colorization compound that is added to the at least one oil component of the present invention is in the range of about 0.1 percent by volume to about 3 percent by volume of the at least one colorization compound per 100 percent by volume of the colorized oil. In another embodiment, the amount of the at least one colorization compound that is added to the at least one oil component of the present invention is in the range of about 0.15 percent by volume to about 2.95 percent by volume, or from about 0.2 percent by volume to about 2.9 percent by volume, or from about 0.25 percent by volume to about 2.85 percent by volume, or from about 0.3 percent by volume to about 2.8 percent by volume, or from about 0.35 percent by volume to about 2.75 percent by volume, or from about 0.4 percent by volume to about 2.7 percent by volume, or from about 0.45 percent by volume to about 2.65 percent by volume, or from about 0.5 percent by volume to about 2.6 percent by volume, or from about 0.55 percent by volume to about 2.55 percent by volume, or from about 0.6 percent by volume to about 2.5 percent by volume, or from about 0.65 percent by volume to about 2.45 percent by volume, or from about 0.7 percent by volume to about 2.4 percent by volume, or from about 0.75 percent by volume to about 2.35 percent by volume, or from about 0.8 percent by volume to about 2.3 percent by volume, or from about 0.85 percent by volume to about 2.25 percent by volume, or from about 0.9 percent by volume to about 2.2 percent by volume, or from about 0.95 percent by volume to about 2.15 percent by volume, or from about 1 percent by volume to about 2.1 percent by volume, or from about 1.05 percent by volume to about 2.05 percent by volume, or from about 1.1 percent by volume to about 2 percent by volume, or from about 1.15 percent by volume to about 1.95 percent by volume, or from about 1.2 percent by volume to about 1.9 percent by volume, or from about 1.25 percent by volume to about 1.85 percent by volume, or from about 1.3 percent by volume to about 1.8 percent by volume, or from about 1.35 percent by volume to about 1.75 percent by volume, or from about 1.4 percent by volume to about 1.7 percent by volume, or from about 1.45 percent by volume to about 1.65 percent by volume, or from about 1.5 percent by volume to about 1.6 percent by volume, or even about 1.55 percent by volume of the at least one colorization compound per 100 percent by volume of the colorized oil.

Given the above, in one embodiment the present invention relates to a colorized oil that comprises at least one oil selected from one or more silicone oils, one or more hydrocarbon oils, or a combination of any two or more thereof having a viscosity in the range of about 500 centipoise to about 1500 centipoise in combination with at least one colorization compound selected from one or more fluorescent acrylic compositions, one or more ultraviolet dyes, or a combination of any two or more thereof, where the at least one colorization compound fluoresces at a wavelength of about 100 nm to about 400 nm. In another embodiment, in the colorized oil of the present invention the at least one oil is selected from at least one silicone oil. In still another embodiment, the colorized oil of the present invention comprises at least one oil is selected from at least one silicone oil having a viscosity of about 1000 centipoise. In still another embodiment, the colorized oil of the present invention comprises from about 0.1 percent by volume to about 3 percent by volume of the at least one colorization compound per 100 percent by volume of the colorized oil. In still another embodiment, the colorized oil of the present invention comprises from about 0.25 percent by volume to about 2.5 percent by volume of the at least one colorization compound per 100 percent by volume of the colorized oil. In still another embodiment, the colorized oil of the present invention comprises from about 0.5 percent by volume to about 2 percent by volume of the at least one colorization compound per 100 percent by volume of the colorized oil. In still another embodiment, the colorized oil of the present invention comprises at least one colorization compound that is a fluorescent acrylic composition that fluoresces at a wavelength of about 315 to about 400 nm. In still another embodiment, the colorized oil of the present invention comprises at least one colorization compound that is a fluorescent acrylic composition that fluoresces at a wavelength of about 365 nm.

In another embodiment, present invention relates to a colorized silicone oil comprising at least one silicone oil, where the at least one silicone oil has a viscosity in the range of about 500 centipoise to about 1500 centipoise, and at least one colorization compound selected from one or more fluorescent acrylic compositions, one or more ultraviolet dyes, or a combination of any two or more thereof, wherein the at least one colorization compound fluoresces at a wavelength of about 100 nm to about 400 nm. In still another embodiment, in the colorized silicone oil of the present invention the at least one silicone oil has a viscosity of about 1000 centipoise. In still another embodiment, the colorized silicone oil of the present invention comprises from about 0.1 percent by volume to about 3 percent by volume of the at least one colorization compound per 100 percent by volume of the colorized silicone oil. In still another embodiment, the colorized silicone oil of the present invention comprises from about 0.25 percent by volume to about 2.5 percent by volume of the at least one colorization compound per 100 percent by volume of the colorized silicone oil. In still another embodiment, the colorized silicone oil of the present invention comprises from about 0.5 percent by volume to about 2 percent by volume of the at least one colorization compound per 100 percent by volume of the colorized silicone oil. In still another embodiment, the colorized silicone oil of the present invention comprises at least one colorization compound that is a fluorescent acrylic composition that fluoresces at a wavelength of about 315 to about 400 nm. In still another embodiment, the colorized silicone oil of the present invention comprises at least one colorization compound that is a fluorescent acrylic composition that fluoresces at a wavelength of about 365 nm.

In another embodiment, present invention relates to a method of calibration and detection of silicone oil in syringe barrels, comprising the steps of: (a) providing a syringe barrel which has an inner surface coated with a silicone oil; (b) obtaining an image with an imaging system of the inner surface of the syringe barrel coated with the silicone oil, wherein the imaging system is capable of detecting silicone oil; and (c) determining from the image obtained from step (b) how the silicone oil is interacting with at least one internal feature present in the syringe barrel. In still another embodiment, the method of the present invention comprises using colorized silicone oil where the colorized silicone oil is formed from a combination of at least one silicone oil having a viscosity in the range of about 500 centipoise to about 1500 centipoise, and at least one colorization compound selected from one or more fluorescent acrylic compositions, one or more ultraviolet dyes, or a combination of any two or more thereof, where the at least one colorization compound fluoresces at a wavelength of about 100 nm to about 400 nm. In still another embodiment, in the colorized silicone oil of the present invention the at least one silicone oil has a viscosity of about 1000 centipoise. In still another embodiment, the colorized silicone oil of the present invention comprises from about 0.1 percent by volume to about 3 percent by volume of the at least one colorization compound per 100 percent by volume of the colorized silicone oil. In still another embodiment, the colorized silicone oil of the present invention comprises from about 0.25 percent by volume to about 2.5 percent by volume of the at least one colorization compound per 100 percent by volume of the colorized silicone oil. In still another embodiment, the colorized silicone oil of the present invention comprises from about 0.5 percent by volume to about 2 percent by volume of the at least one colorization compound per 100 percent by volume of the colorized silicone oil. In still another embodiment, the colorized silicone oil of the present invention comprises at least one colorization compound that is a fluorescent acrylic composition that fluoresces at a wavelength of about 315 to about 400 nm. In still another embodiment, the colorized silicone oil of the present invention comprises at least one colorization compound that is a fluorescent acrylic composition that fluoresces at a wavelength of about 365 nm.

While this disclosure has been described as having exemplary designs, the present disclosure can be further modified within the spirit and scope of this disclosure. This application is therefore intended to cover any variations, uses, or adaptations of the disclosure using its general principles. Further, this application is intended to cover such departures from the present disclosure that are known or customarily practiced in the art to which this disclosure pertains and which fall within the limits of the appended claims.

## Claims

1. A colorized oil comprising:
at least one oil selected from one or more silicone oils, one or more hydrocarbon oils, or a combination of any two or more thereof, the at least one oil having a viscosity in the range of about 500 centipoise to about 1500 centipoise; and
at least one colorization compound selected from one or more fluorescent acrylic compositions, one or more ultraviolet dyes, or a combination of any two or more thereof, wherein the at least one colorization compound fluoresces at a wavelength of about 100 nm to about 400 nm.

2. The colorized oil of claim 1, wherein the at least one oil is selected from at least one silicone oil.

3. The colorized oil of any of claims 1-2, wherein the colorized oil comprises from about 0.1 percent by volume to about 3 percent by volume of the at least one colorization compound per 100 percent by volume of the colorized oil.

4. The colorized oil of any of claims 1-3, wherein the colorized oil comprises from about 0.5 percent by volume to about 2 percent by volume of the at least one colorization compound per 100 percent by volume of the colorized oil.

5. The colorized oil of any of claims 1-4, wherein the at least one colorization compound is a fluorescent acrylic composition that fluoresces at a wavelength of about 315 to about 400 nm.

6. A colorized silicone oil comprising:
at least one silicone oil, the at least one silicone oil having a viscosity in the range of about 500 centipoise to about 1500 centipoise; and
at least one colorization compound selected from one or more fluorescent acrylic compositions, one or more ultraviolet dyes, or a combination of any two or more thereof, wherein the at least one colorization compound fluoresces at a wavelength of about 100 nm to about 400 nm.

7. The colorized silicone oil of claim 6, wherein the at least one silicone oil has a viscosity of about 1000 centipoise.

8. The colorized silicone oil of any of claim 6 or 7, wherein the colorized silicone oil comprises from about 0.1 percent by volume to about 3 percent by volume of the at least one colorization compound per 100 percent by volume of the colorized silicone oil.

9. The colorized silicone oil of any of claims 6-8, wherein the colorized silicone oil comprises from about 0.5 percent by volume to about 2 percent by volume of the at least one colorization compound per 100 percent by volume of the colorized silicone oil.

10. The colorized silicone oil of any of claims 6-9, wherein the at least one colorization compound is a fluorescent acrylic composition that fluoresces at a wavelength of about 315 to about 400 nm.

11. A method of calibration and detection of colorized silicone oil in syringe barrels, comprising the steps of:
(a) providing a syringe barrel which has an inner surface coated with a colorized silicone oil;
(b) obtaining an image with an imaging system of the inner surface of the syringe barrel coated with the colorized silicone oil, wherein the imaging system is capable of detecting colorized silicone oil; and
(c) determining from the image obtained from Step (b) how the colorized silicone oil is interacting with at least one internal feature present in the syringe barrel.

12. The method of claim 11, wherein the colorized silicone oil comprises:
at least one silicone oil, the at least one silicone oil having a viscosity in the range of about 500 centipoise to about 1500 centipoise; and
at least one colorization compound selected from one or more fluorescent acrylic compositions, one or more ultraviolet dyes, or a combination of any two or more thereof, wherein the at least one colorization compound fluoresces at a wavelength of about 100 nm to about 400 nm.

13. The method of any of claims 11-12, wherein the colorized silicone oil comprises from about 0.1 percent by volume to about 3 percent by volume of the at least one colorization compound per 100 percent by volume of the colorized silicone oil.

14. The method of any of claims 11-13, wherein the colorized silicone oil comprises from about 0.5 percent by volume to about 2 percent by volume of the at least one colorization compound per 100 percent by volume of the colorized silicone oil.

15. The method of any of claims 11-14, wherein the at least one colorization compound is a fluorescent acrylic composition that fluoresces at a wavelength of about 315 to about 400 nm.
